# EUROPEAN PATENT SPECIFICATION

(11) **EP 2 585 087 B1**
(45) Date of publication and mention of the grant of the patent: **13.12.2017**
(21) Application number: 11795242.4
(22) Date of filing: 16.06.2011
(51) Int. Cl.: A61K 36/45, A61K 8/97, A61P 31/04, A61P 31/10, A61Q 11/02, C12R 1/865

(54) **A PREPARATION FOR BALANCING THE COMPOSITION OF THE ORAL MICROBIAL FLORA**
PRÄPARAT ZUM AUSGLEICHEN DER ZUSAMMENSETZUNG DER MIKROBIELLEN MUNDFLORA
PRÉPARATION PERMETTANT D'ÉQUILIBRER LA COMPOSITION DE LA FLORE MICROBIENNE ORALE

(30) Priority: 18.06.2010 FI 20105710
(43) Date of publication of application: 01.05.2013
(73) Proprietor: Pärnänen, Pirjo, 01420 Vantaa (FI)
(72) Inventor: Pärnänen, Pirjo, 01420 Vantaa (FI)
(74) Representative: Seppo Laine Oy
(86) International application number: PCT/FI2011/050572
(87) International publication number: WO 2011/157898

(56) References cited:
- WO-A1-2009/018470
- WO-A2-00/16732
- FI-U1- 8 195
- LV-B- 14 031
- US-A- 4 857 327
- NOHYNEK L J ET AL: "Berry Phenolics: Antimicrobial Properties and Mechanisms of Action Against Severe Human Pathogens", NUTRITION AND CANCER, TAYLOR & FRANCIS GROUP, GB, vol. 54, no. 1, 1 January 2006 (2006-01-01), pages 18-32, XP003023871, ISSN: 0163-5581, DOI: 10.1207/S15327914NC5401_4
- KAISU RIIHINEN ET AL: "Antiaggregation potential of berry fractions against pairs of Streptococcus mutans with Fusobacterium nucleatum or Actinomyces naeslundii", PHYTOTHERAPY RESEARCH, vol. 25, no. 1, 8 July 2010 (2010-07-08), pages 81-87, XP055101590, ISSN: 0951-418X, DOI: 10.1002/ptr.3228
- MARKO TOIVANEN ET AL: "Screening of binding activity of Streptococcus pneumoniae, Streptococcus agalactiae and Streptococcus suis to berries and juices", PHYTOTHERAPY RESEARCH, vol. 24, no. S1, 16 July 2009 (2009-07-16) , pages S95-S101, XP055101600, ISSN: 0951-418X, DOI: 10.1002/ptr.2939
- A. VISTI ET AL.: 'Preparation of fermentable lingonberry juice through removal of benzoic acid by Saccharomyces cerevisiae yeast' FOOD RESEARCH INTERNATIONAL vol. 36, no. 6, 2003, pages 597 - 602, XP055073540

## Description

### Field of the Invention

The present invention relates to a preparation obtained using lingonberry juice. Sucking tablets manufactured from lingonberry juice, from which sugars have been removed and which has been dried into powder, inhibit growth of undesirable fungi and bacteria of human oral microbial flora. Instead, the preparation favours growth of the beneficial *Lactobacillus* strains in the mouth.

### Background of the Invention

The normal oral microbial flora is usually a mixture of commensal and pathogenic microbes including also *Streptococcus* bacteria, *Candida* yeasts and *Lactobacilli.* The last mentioned are believed to have a protective role in balancing the oral flora probably by competing of nutrients and living space with pathogens and the opportunistic *Candida. Streptococci* are involved in causing dental caries. Saliva is the first line of defence against infection and it acts through microbicidal enzymes and leukocytes. When the secretion of saliva decreases, its microbicidal and spooling effect diminishes and this makes the mucosa more vulnerable to infection. Also immunocompromizing diseases such as diabetes mellitus and cancer lower the resistance against infection. In addition, medications used in asthma, psychiatric diseases or hypertension can make teeth and oral mucosa more vulnerable to infections caused by pathogenic bacteria or opportunistic yeasts. A newborn with an incomplete immune defence and older population with ill fitting dental prostheses, medications and high age causing immunosuppression belong to the group of higher infection risk.

*Candida albicans* (*C. albicans*) is the most commonly found *Candida* species in the oral cavity. Other species isolated orally are in order of prevalence: *Candida dubliniensis* (*C. dubliniensis*), *Candida glabrata* (*C. glabrata*), *Candida parapsilosis* (*C. parapsilosis*), *Candida tropicalis* (*C. tropicalis*) and *Candida krusei* (*C. krusei*)*. C. parapsilosis* is often found in invasive candidosis. Resistance against the most commonly used fungal drugs azoles have been reported increasingly for the species *C. albicans* and *C. glabrata* is innately resistant to azoles, and efforts are made to find an efficient, safe and inexpensive antifungal agent.

Anthocyanins are found in plants and they have many biological activities. Anthocyanins are soluble in water, but insoluble in non-hydroxylic solvents such as ether, acetone, chloroform and benzene (Geissman, 1962). Lingonberry (*Vaccinium vitis-idaea*) is especially rich in idaein (cyanidine-3-galactoside). Lingonberry is generally regarded as safe, and a study of Laitinen *et al.* (2004) showed that lingonberry had no effect on the permeability of drugs across Caco-2 cell monolayers.

In traditional medicine lingonberry is used as an antifungal agent, but only a few studies of this effect against *C. albicans* have been conducted, and the active agent against it has been proposed to be the anthocyanin idaein, organic acids, 6-benzyl-D-glucose or some other unidentified substances. Lingonberries contain high amounts of the anthocyanins cyanidin-3-galactoside (88%), cyanidin-3-arabinoside (10.6%) and cyanidin-3-glucoside (1.4%) (Andersen, 1985).

Rauha *et al.* (1999) have prepared an extract of lingonberry with 70% aqueous acetone, rotary evaporated and sugars removed. The extract manufactured this way had no inhibitory effect on *Candida albicans* ATCC 10231 growth. Újváry *et al.* (1961) used ethanol as extracting solvent and this extract had an inhibitory effect on *C. albicans* growth with dilutions under 1:4. Andersen (1985), Deubert *et al.* (1987) and Liu *et al.* (2004) used acidified ethanol in the extraction of anthocyanins from lingonberry. Antifungal properties of cranberry juice have also been studied and it had no apparent effect on *C. albicans* (Swartz and Medrek, 1968) and this study proposes that the mild fungistatic effect is caused by benzoic acid.

Various methods have been used to remove sugars from berry and fruit juices: chromatography (Walker et al. AU 199522726 B2; cranberry, bilberry, European cranberry and blueberry), dialyzation (WO 00/16732; cranberry), and filtration methods (WO 2009/018470 A1; cranberry). Fermentation has been long used for removing sugars, for example Frerichs (1914) manufactured vegetable extracts by alcoholic fermentation of vegetable juices. Anaerobic fermentation with 2.5 g/L of *S. cerevisiae* has been used for recovering polyphenolics and removing sugars from muscadine grape wine byproduct pomace (Cardona *et al.* 2009). This is a much too low amount of *S. cerevisiae* for lingonberry to fermentate. Visti *et al.* (2003) pretreated lingonberry juice for 10 min with 0.06% *S. cerevisiae* to remove excess benzoic acid, and then fermented the juice for 7 d with 15-20% (w/w) S. *cerevisiae* resulting in a 59% drop in sugar content and an alcohol content of 3.5%. No antimicrobial tests were conducted in this study.

The present inventor has for a few years studied lingonberry juice as an antifungal agent. Finnish Utility Model No. 8195 describes tests in which lingonberry juice concentrate is studied *in vitro* for its ability to inhibit the growth of *Candida* yeasts, *Streptococci* and *Lactobacilli* of oral microflora. Based on the results, it was suggested that lingonberry juice would be useful in balancing the oral microbial flora. However, lingonberry juice contains plenty of sugars, and in *in vivo* tests the juice turned out to be unsuitable for this purpose. Consequently, a need still exists for an effective preparation suitable for balancing the microbial flora in mouth.

### Summary of the Invention

The present inventor has now found that for manufacturing a suitable lingonberry juice-based preparation, which is effective against undesirable fungi and bacteria of oral cavity, the juice should first be treated so that it is possible to dry the juice to a dry cake from which tablets can be pressed. A suitable treatment turned out to be fermentation with a sufficient amount of *Saccharomyces cerevisiae* yeast. The preparation manufactured from the fermented juice is substantially free of sugars. The preparation is preferably in the form of sucking tablets.

Consequently, the primary object of the present invention is a lingonberry preparation which is obtainable by a process which comprises the steps of
a) fermenting cold-pressed lingonberry juice with *Saccharomyces cerevisiae,*
b) removing yeast cells from the fermented juice,
c) drying the juice to obtain a substantially dry cake,
d) milling the cake obtained into powder, and
e) pressing the powder into sucking tablets,
for use in inhibiting the growth of undesirable yeasts and bacteria and promoting the growth of probiotic bacteria in mouth. In a specific embodiment the undesirable yeasts are of the genus *Candida,* and the undesirable bacteria are *Streptococci.* The probiotic bacteria whose growth is promoted are lactobacilli.

### Brief Description of Drawings

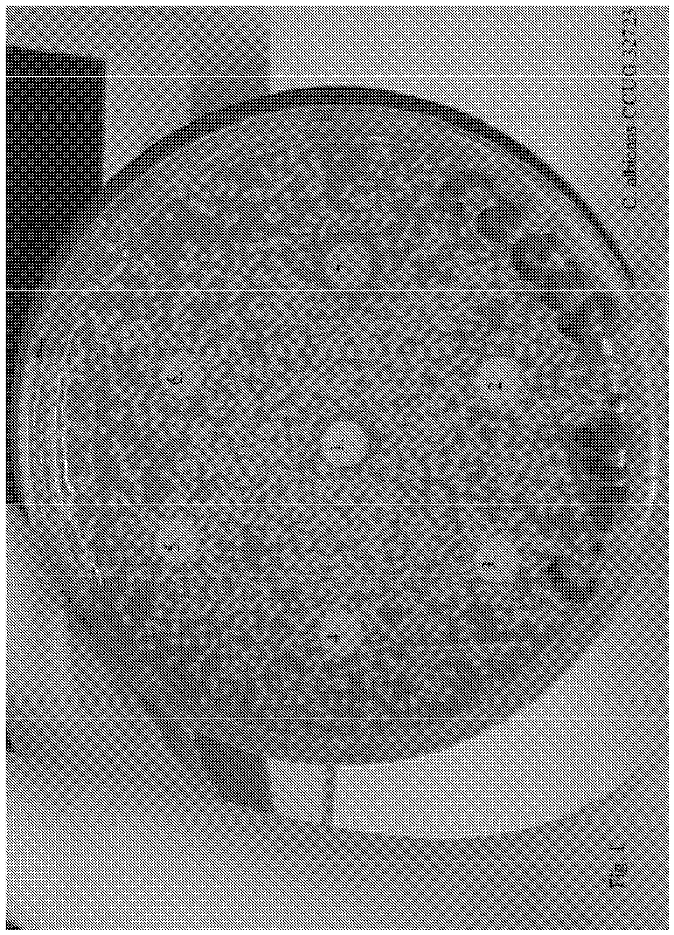

### Figure 1. Agar-plate-test for C. albicans CCUG 32723.

Disk 1: 0.2 % chlorhexidine; Disk 2: 0.9% NaCl, Disk 3: juice preparation, non-concentrated; Disk 4: juice preparation, 5 x concentrated; Disk 5: juice preparation, 10 x concentrated; Disk 6: juice preparation 25 x concentrated; Disk 7: juice preparation, 50 x concentrated.

### Figure 2. MIC-lines for C. albicans CCUG 32723.

Line 0: *C. albicans* with 0.9 % NaCl; Line 1: *C. albicans* with 50 x concentrated juice preparation; Line 2: *C. albicans* with 25 x concentrated juice preparation; Line 3: *C. albicans* with 10 x concentrated juice preparation; Line 4: *C. albicans* with 5 x concentrated juice preparation; Line 5: *C. albicans* with 0.2 % chlorhexidine.

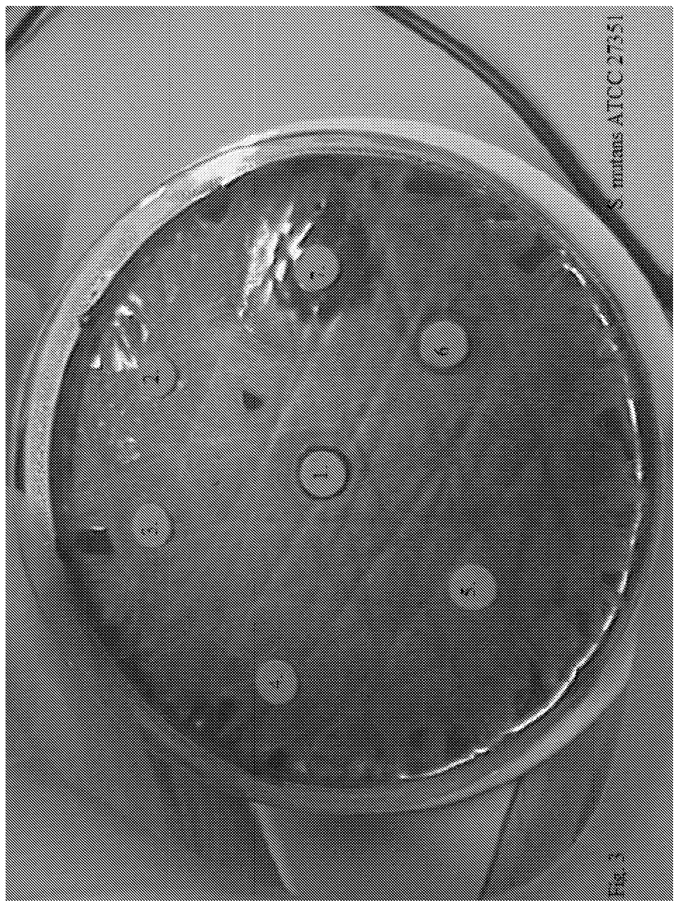

### Figure 3. Agar-plate-test for S. mutans ATCC 27351.

Disk 1: 0.2 % chlorhexidine; Disk 2: 0.9% NaCl, Disk 3: juice preparation, non-concentrated; Disk 4: juice preparation, 5x concentrated; Disk 5: juice preparation, 10 x concentrated; Disk 6: juice preparation 25 x concentrated; Disk 7: juice preparation, 50 x concentrated.

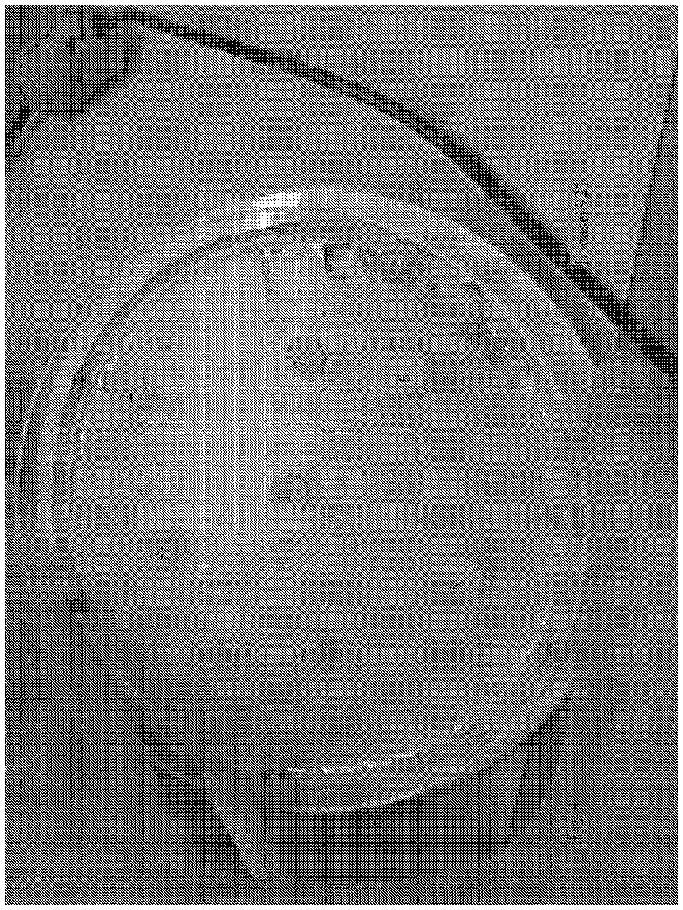

### Figure 4. Agar-plate-test for L. casei 921.

Disk 1:0.2 % chlorhexidine; Disk 2: 0.9% NaCl, Disk 3: juice preparation, non-concentrated; Disk 4: juice preparation, 5x concentrated; Disk 5: juice preparation, 10 x concentrated; Disk 6: juice preparation 25 x concentrated; Disk 7: juice preparation, 50 x concentrated.

### Detailed Description of the Invention

A study was set out to test the effect of lingonberries on the growth of commensal human oral flora inhabitants: *Candida, Streptococci* and *Lactobacilli.* Concentrated lingonberry juice was tested *in vitro* on 12 different *Candida* strains, 4 *Streptococcus* strains and two *Lactobacillus* strains. This study tested also the effect of addition of extra idaein chloride on the growth of these organisms. Idaein chloride is soluble in water and mild hydrochloric acid.

*In vivo* tests with sucking tablets manufactured from lyophilized lingonberry juice were also conducted. The MIC (minimal inhibitory concentration) was determined by a modified microtiter assay *in vitro.* Generally 25x to 50x concentrated (in some strains even in lower concentrations) lingonberry juice preparation inhibited the growth of all of the *Candida* and three of the *Streptococcus* strains. There was no growth inhibition of the *Lactobacilli* tested. *In vivo* the sucking tablets inhibited *Candida* and *S. mutans* growth, but instead favoured *Lactobacilli* growth. Lingonberry seems a promising inexpensive anticandidal and antistreptococcal agent. It could thus be used for balancing the oral microbial flora.

While cranberry (*Vaccinium macrocarpum*) belongs to the same genus as lingonberry (*Vacciniun vitis-idaea*), these berries have different chemical compositions. For instance, the benzoic acid concentration of lingonberry (0.22%; Griebel, 1910) is much higher than that of cranberry (in average 0.065%; Mason, 1905; Radin, 1914; Blatherwick & Long, 1923; Nelson 1927). Therefore, fermentation of cranberry juice is much easier than fermentation of lingonberry juice. As shown in the comparative test below, there was a great difference in the amount of yeast needed for the process to succeed. The fermentation technology of lingonberry thus turned out to be very challenging.

The antimicrobial spectrum is also different in cranberry compared to lingonberry. In an *in vitro* study described below it was shown that cranberry juice had no inhibitory effect on *Candida* and *Streptococcus,* but lingonberry juice had an inhibitory effect on these microbes. In addition, lingonberry juice did not inhibit *Lactobacillus.*

The manufacturing method described in this specification is carried out using as starting material cold-pressed lingonberry juice. Sugars are removed from the juice by fermenting it anaerobically with ordinary baker's yeast (*Saccharomyces cerevisiae*), which was chosen because it is generally regarded as safe. Fermentation is also technically more simple than other methods to remove sugars. The amount of the yeast needed is as high as 27 - 33 grams of lyophilized yeast per ½ litres of juice. Fermentation is let go up to the end of fermentation, usually for about 7 days. The yeast cells are then removed from the fermented juice, preferably by filtering the juice. Subsequently, the filtered juice is dried to obtain a substantially dry cake. Preferably, the juice is dried by lyophilization (freeze-drying). Alternatively, spray-drying may be used. Appropriate moisture content for the lyophilizate is 1- 5 % (w/w). Subsequently, the lyophilizate is milled, or ground in a mortar, and the powder obtained is pressed to sucking tablets. 5% (w/v) of magnesium stearate in acetone may be used as a lubricant in the tablet pressing device. A powder with appropriate moisture content may be pressed without additives. Powdered or granulated mannitol, for instance, may be used as an additive in the pressing step. Other suitable additives may also be used. However, it should be noted that the additive used should not promote the growth of yeasts. Therefore, sugars may not be useful for that purpose. Instead, xylitol or any other artificial sweetener may obviously be used as an appropriate additive. The additive prevents the pressed tablet from melting to an amorphic form during storage.

The fermentation process, by removing sugars, made it possible to freeze-dry the lingonberry juice to a dry cake from which tablets could be pressed. It should be noted that the preparation with sugars left in Finnish Utility Model No. 8195 could not be lyophilized to powder, but the material bubbled in the lyophilizer, and the product was a sticky concentrated mass, from which it was technically impossible to press tablets. In addition, said product could not be concentrated to the degree and to the anticandidal and antistreptococcal level, which is necessary for the product of the present invention. Technically, fermentation is also a much more inexpensive method compared to column purification, which is a benefit in production costs.

Furthermore, in a clinical trial described below it is shown that the material according to the FI Utility Model No. 8195 (mouthrinse) surprisingly had *in vivo* an effect of dramatic and unexpected increase in *Candida* growth. This was not expected from the *in vitro* studies performed with the same mouthrinse. To this background the claimed lingonberry preparation formula for use according to the present invention with sugars removed is substantially different from the lingonberry mouthrinse according to FI Utility Model 8195.

Examples are shown below of each test category; results of all strains are gathered into the Tables below, wherein
Table 1. shows MIC-lines for *Candida,*
Table 2. MIC colony counting results for *Candida,*
Table 3. results of the agar-plate-tests, and
Table 4. the *in vivo* test results.

### Experimental

### Materials and Methods

**Preparation of lingonberry juice concentrate and lingonberry sucking tablets:**
Commercial cold pressed and pasteurised lingonberry juice without added sugar (Aten Marja-Aitta, Puumala, Finland) was used as starting material. Natural sugars of the juice were removed by fermenting the juice with *S. cerevisiae.* Briefly, 33 g of lyophilized commercial *S. cerevisiae* preparation containing E 491 emulsifying agent (Ravintoraisio, Raisio, Finland) were added into ½ litres of juice and fermentation was carried out for about 7 days, i.e. up to the end of fermentation. Alcohol content of the fermented juice was 11%. Subsequently, yeast cells were removed from the fermented juice by filtering it with Vinoferm filtering appliance through silica disks (coarseness: fine), and finally through Millipore Stericup and Steristop filters (0.22 µm) to remove the rest of the yeast cells. After that the filtered juice was lyophilized for 5 days to obtain a substantially dry cake. For the *in vitro* tests 5 x (0.35 g/ml), 10 x (0.7 mg/ ml), 25 x (1.75 g/ml) and 50 x (3.5 g/ml) concentrates of the juice preparation were prepared by dissolving the lyophilizate into 0.9 % NaCl.

The moisture content of the lyophilizate was under 5.4 %. Subsequently, the lyophilizate was ground in a mortar, and for use in the *in vivo* tests 3.5 g of the powder obtained was pressed to sucking tablets without additives. Briefly, 3.5 g of lyophilised juice preparation was weighed and added to the Mg-stearate-lubricated tablet pressing device and by hand force pressed to a tablet form. The tablets were stored at + 8 °C in sealed plastic bags to prevent wetting.

### Liquid chromatography and mass analysis:

In order to determine the composition of lingonberry juice in general, the content of anthocyanins and acylated anthocyanins, katechins, flavonoid glycosides, phenolic acids and their conjugates of non-concentrated juice, lyophilized juice, and juice incubated for 1 hour in acidified ethanol (85 ml ethanol with 15ml HCl (1.5 M)) and then 0.45 µm filtered resulting extract were analyzed by liquid chromatography. In addition, LC-pESI-IT-MS (liquid chromatography electrospray ionization ion trap mass spectrophotometry) was used in identification of anthocyanins. The polarity of the electrospray ionization was positive. The analyses were performed with parallel samples.

### MIC:

The tested *Candida* strains were: *C. albicans* ATCC 32723, *C. albicans* B 1134, *C. dubliniensis* Cd3, *C. dubliniensis* Cd4, *C. parapsilosis* Cp2, *C. parapsilosis* Cp3, *C. glabrata* ATCC 32725, *C. glabrata* G212, *C. krusei* ATCC 6268, *C. krusei* D206B, *C. tropicalis* ATCC 750 and *C. tropicalis* D213. The yeasts were grown on SDA-agar (Sabouraud dextrose agar, Lab M, Lancashire, UK) at 37 °C. From each plate *Candida* colonies were taken into Sabouraud liquid and diluted with 0.9% NaCl to a cell density of 10³ cfu/ml. 90 µl of this *Candida* dilution was incubated with 10 µl of 5, 10, 25 and 50x concentrated lingonberry juice preparate for 5 minutes, then centrifuged for 1 min at 13000 rpm, supernatants were removed, 100 µl of 0.9 % NaCl was added on each pellet, suspended and lines were drawn on SDA-agar with a wooden stick. The plates were incubated for 24 h at 37 °C. As a positive control 90 µl *Candida* with 10 µl of 0.2 % chlorhexidine and as a negative control 90 µl of *Candida* with 10 µl of 0.9 % NaCl were used. Also the effect of benzoic acid was tested. Lingonberries contain naturally 70-200 mg of benzoic acid per 100g, and the range of 0.65 mg/ml - 65 mg/ml was tested.

In addition, 100 µl of the 25 x, 50 x concentrated lingonberry juice preparate incubated with *Candida* for 5 min as described previously were cultivated on SDA, incubated overnight at 37 °C, and colonies were counted and compared to the *Candida* controls (not treated with lingonberry).

### Agar-plate tests:

10³ cfu of *Candida* suspension was prepared and was applied onto an SDA-agar plate, spread evenly with a glass triangle and 6 mm paper discs were added onto the plate. 10 µl of the lingonberry juice preparate and its 5, 10, 25 and 50 x concentrates, or pure idaein chloride (Extrasynthese, Genay, France) were applied onto the discs. The concentrations of idaein chloride tested were: 1, 0.1, 0.01 and 0.001 mg/ml. The sole effect of acidity on the growth of *Candida* were tested with 10 µl of 1.5 M HCl (Idaein, benzoic acid, and HCl data not shown).

The tested *Lactobacillus* strains were: *L. rhamnosus* 5.1.a and *L. casei* 921. The *Lactobacilli* included in this study are the most commonly found species from the oral cavity. The *Lactobacilli* were grown on MRS-agar (De Man, Rogosa and Sharpe; Lab M, Lancashire, UK) in a 5 % CO₂ atmosphere and colonies were taken into 0.9 % NaCl and three dilutions were made from this (McFarland -2 density) stock. The dilutions were spread evenly on MRS-agar with a glass triangle. 10 µl of 5, 10, 25 and 50 x concentrated lingonberry juice preparate were applied onto paper discs.

The tested streptococcal strains were: *S. mutans* ATCC MT 81482(c), *S. mutans* ATCC 27351, *S. sanguinis* ATCC 10556 and *S. salivarius* ATCC 13419. The *Streptococci* were grown on blood agar in a CO₂ atmosphere and colonies were suspended into 0.9 % NaCl and three dilutions were made from this (McFarland -2 density) stock. The dilutions were spread evenly on blood-agar with a glass triangle. 10 µl of 5, 10, 25 and 50 x concentrated lingonberry juice were applied onto paper discs as above.

In all agar-plate tests as a negative control 10 µl of 0.9 % NaCl and as a positive control 10 µl of 0.2 % chlorhexidine were used. The plates were incubated overnight at 37 °C, *Lactobacilli* and *Streptococci* in a 5 % CO₂-atmosphere.

### In vivo tests:

10 adult test subjects were divided into two groups: those who had under 10⁴ cfu/ml *Candida* growth in stimulated saliva or those with over 10⁴ cfu/ml *Candida.* Participants with higher yeast count used three sucking tablets/day and subjects with lower yeast counts twice daily. Both groups used the sucking tablets for 10 days. The subjects were advised to let the sucking tablet dissolve slowly in the mouth, which took approximately 6 minutes/ tablet. The initial and post-treatment *S. mutans, Lactobacilli* and *Candida* levels were measured by commercial tests according to manufacturer's instructions (Dentocult ®SM Strip mutans, Dentocult ® LB and Dentocult® CA tests, respectively; Orion Diagnostica, Espoo, Finland). The *Candida* levels were also counted from 100 microlitres of stimulated saliva cultivated on SDA at 37 °C overnight. *Candida* were identified by cultivating on chromagar plates and *C. albicans* further distinguished from *C. dubliniensis* with Bichro-Dubli Fumouze test (Fumouze Diagnostics, France).

### Results

### Liquid chromatography and mass analysis:

Total benzoic acid concentration was lowest in the non-concentrated juice and highest in lingonberry extract. Three additional spikes were detected in the chromatogram, which resembled the UV spectrum of benzoic acid, and they might be sugar conjugates of benzoic acid. In the juice and the lyophilized juice three different catechins were detected, but these were not further analyzed, and were quantitated as + catechin. The qualitative analysis of the anthocyanins revealed that the main (85%) anthocyanin component of all the samples was cyanidin-3-glucoside or cyanidin-3-galactoside. Compared to the literature (Andersen, 1985) it is most probably cyanidin-3-galactoside. The other identified anthocyanin (10%) was cyanidin-3-arabinoside. The third spike (5 %) remained unidentified by these methods. Results are not shown.

### MIC:

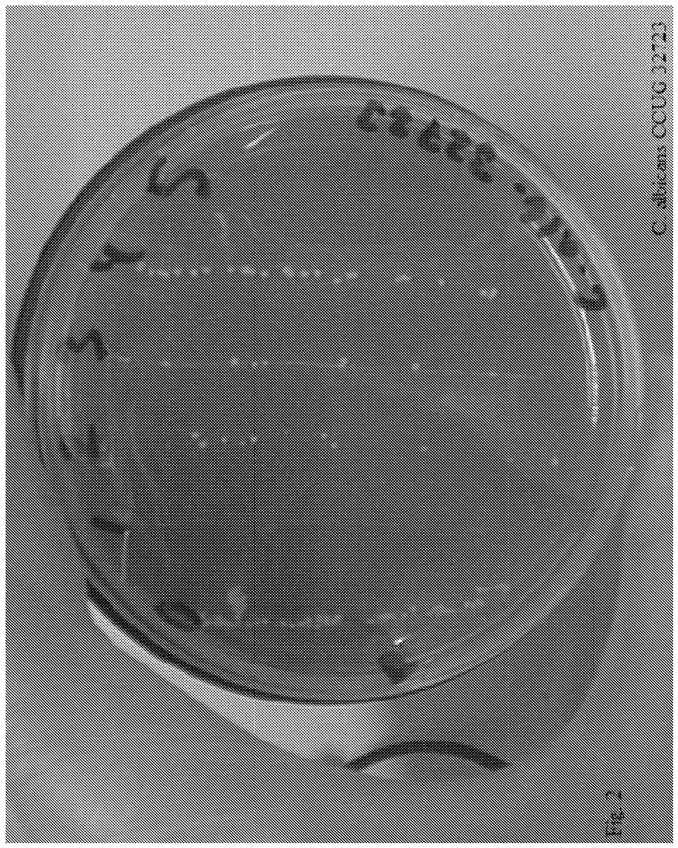

Inhibition of *Candida* growth by the extract was seen as no growth on the line struck compared to the positive control with growing colonies. The lingonberry juice concentrate inhibited the growth of all the *Candida* tested at concentrations from 25 to 50 x, in some strains even at lower concentrations (Figure 2. and Table 1.). There was no growth with chlorhexidine. The control *Candida,* 25 x and 50 x concentrated lingonberry preparation treated *Candida* cfus are seen in Table 2. The 25 x concentration lowers approximately to half the initial *Candida* colony count, the 50 x concentrate inhibits *Candida* growth totally after a 5 minute treatment *in vitro.*

**Table 1. MIC-lines for Candida.**

| Strains | **0.** | 1. | 2. | 3. | 4. | 5. |
|---|---|---|---|---|---|---|
| 32723 | + | - | + | + | + | - |
| B1134 | + | - | + | + | + | - |
| Cd3 | + | - | + | + | + | - |
| Cd4 | + | - | + | + | + | - |
| Cp2 | + | - | + | + | + | - |
| Cp3 | + | - | + | + | + | - |
| 32725 | + | - | + | + | + | - |
| G212 | + | - | + | + | + | - |
| 6258 | + | - | + | + | + | - |
| D206B | + | - | + | + | + | - |
| 750T | + | - | + | + | + | - |
| D213 | + | - | + | + | + | - |

| | | | | | | |
|---|---|---|---|---|---|---|
| (+) colony growth (-) no colony growth | | | | | | |

**Table 2. Candida colony counts. Cfus/ 100 µl are indicated after 5 min treatment with 25x and 50x concentrated lingonberry juice preparate compared to the untreated Candida.**

| Strains | *Candida* control | 25 x | 50 x |
|---|---|---|---|
| 32723 | 812 | 257 | 0 |
| B1134 | 618 | 438 | 0 |
| Cd3 | 320 | 280 | 0 |
| Cd4 | 226 | 274 | 0 |
| 32725 | 466 | 104 | 0 |
| G212 | 420 | 50 | 0 |
| Cp2 | 364 | 168 | 11 |
| Cp3 | 172 | 105 | 13 |
| 62258 | 148 | 21 | 0 |
| D206B | 588 | 178 | 3 |
| 750T | 388 | 400 | 0 |
| D213 | 17 | 27 | 0 |

### Agar-plate tests:

The inhibitory effect of the lingonberry on the growth of the tested species were seen as growth inhibitory zones around the paper discs. 10 x -50 x concentrates of the juice inhibited growth of all the *Candida* strains tested which could be seen as inhibitory rings around the paper discs (Table 3.). Sole idaein chloride seemed not to have any inhibitory effect on the growth of *Candida.* 0.9% NaCl had no inhibitory effect, HCl inhibited slightly and chlorhexidine inhibited totally, as expected, the growth of the *Candida.*

The growth of three strains of *Streptococci* was inhibited with the 25x and 50 x lingonberry juice preparate. *S. sanguinis* showed least inhibition, but a slight inhibitory effect could be seen with the 50 x concentrate. The concentrates had no inhibitory effect on *Lactobacilli.*

**Table 3. Agar-plate tests. Inhibition zones around discs are indicated in millimetres.**

| Strains | 0.2 % CHx | 50x | 25x | 10x | 5x | not conc. | 0.9 % NaCl |
|---|---|---|---|---|---|---|---|
| 32723 | 8 | 9 | - | - | - | - | - |
| B1134 | 7 | 8 | - | - | - | - | - |
| Cd3 | 8 | 9 | - | - | - | - | - |
| Cd4 | 8 | 8 | - | - | - | - | - |
| 32725 | 8 | 9 | - | - | - | - | - |
| G212 | 8 | 8 | - | - | - | - | - |
| Cp2 | 11 | 7 | - | - | - | - | - |
| Cp3 | 15 | 11 | - | - | - | - | - |
| 6258 | 7 | 7 | - | - | - | - | - |
| D206B | 9 | 8 | - | - | - | - | - |
| 750T | 11 | 9 | - | - | - | - | - |
| D213 | 10 | 8 | - | - | - | - | - |
| 27351 | 8 | 16 | 9 | - | - | - | - |
| 81842 | 7 | 20 | 11 | - | - | - | - |
| 10556 | 10 | (7) | - | - | - | - | - |
| 13419 | 10 | 22 | 9 | - | - | - | - |
| 921 | - | - | - | - | - | - | - |
| 5.1.a | - | - | - | - | - | - | - |

### In vivo tests:

The amount of *Candida* diminished in 6 study subjects out of ten, in three there was an insignificant rise in *Candida* cfu and in one the levels remained unchanged. The *Lactobacilli* levels rose as expected in all study subjects. *S. mutans* levels diminished in 4 out of ten, stayed unchanged in 5 out of ten, and rose in only one subject. The results are shown in Table 4.

**Table 4. In vivo sucking tablet 10 day test results. Initial/ 10 day values indicated in each category.**

| Test subject number | *Candida* cfu/ 100 µl (SDA) | Dentocult ®LB (*Lactobacilli*) count | Dentocult ®CA (*Candida*)*count* | Dentocult® SM Strip mutans (*S*. *mutans*)*scale* 1-3 |
|---|---|---|---|---|
| 1. | 107/ 20 | 10⁴/ 10⁴ | 10³/ 10³ | 3/2 |
| 2. | 227/364 | 10⁴/ 10⁶ | 10⁴/ 10⁴ | 2/3 |
| 3. | 623/480 | 10⁴/ 10⁶ | 10⁴/ 10⁴ | 2/2 |
| 4. | 535/423 | 10⁴/ 10⁶ | 10⁵/ 10⁴ | 3/2 |
| 5. | 347/110 | 10⁵/ 10⁶ | 10⁴/ 10³ | 3/3 |
| 6. | 90/225 | 10⁵/ 10⁶ | 10⁴/ 10⁴ | 2/2 |
| 7. | 77/62 | 10⁴/ 10⁶ | 10³/ 10³ | 3/2 |
| 8. | 18/6 | 10³/ 10⁴ | 10³/ 10³ | 1/0 |
| 9. | 2/22 | 10³/ 10⁴ | 10³/ 10³ | 1/1 |
| 10. | 80/93 | 10³/ 10⁴ | 10³/ 10³ | 1/1 |

### Comparative tests concerning the characteristics of cranberry and lingonberry

### Fermentability of cranberry and lingonberry juices

Lingonberry (*Vaccinium vitis idaea*) juice is not fermentable because of the high benzoic acid content (0.22 %), which is particularly high in lingonberries compared to 0.065% of cranberries (*Vaccinium macrocarpum*). The difference in fermentation of the two *Vaccinium* berries was found using the following test:
Cold-pressed lingonberry and cold-pressed cranberry juices were compared by adding 1-33 g of freeze-dried *Saccharomyces cerevisiae* to 0.5 1 of juice and left to ferment anaeobically in room temperature. Compared to cold-pressed cranberry juice lingonberry juice needed approximately 30 times more *Saccharomyces* yeast (27-33 g) to start and produce the fermentation in 7 d. Cranberry juice needed only 1-5 g of freeze-dried *Saccharomyces cerevisiae* to ferment.

### Antimicrobial effect of cranberry and lingonberry

The antimicrobial spectrum is different in cranberry compared to lingonberry. In an *in vitro* study the inhibitory effect of 10 x concentrated cold-pressed and 10 x concentrated cold-pressed fermented cranberry juice were tested on *Candida albicans* ATCC 32723, *Lactobacillus rhamnosus* and *Streptococcus mutans* ATCC 81482. Cranberry had no inhibitory effect on these microbes, whereas 10 x concentrated cold-pressed and fermented 10 x concentrated cold-pressed lingonberry juice had an inhibitory effect on *Candida albicans* ATCC 32723 and *Streptococcus mutans* ATCC 81482, but did not inhibit *Lactobacillus rhamnosus.*

### In vivo test using concentrated lingonberry juice mouthrinse according to FI Utility Model No. 8195

Removing of sugars from the lingonberry juice was necessary to obtain the anticandidal inhibitory effect *in vivo.* A clinical trial was performed with concentrated lingonberry juice mouthrinse according to FI Utility Model No. 8195, which contained all the natural sugars of the cold-pressed juice. Ten milliliters of the mouthrinse was given twice daily to 60 study subjects for 20 days. The mouthrinse did inhibit *Streptococcus mutans* growth but did not inhibit the growth of *Lactobacilli.* Surprisingly, the mouthrinse had *in vivo* an effect of dramatic and unexpected increase in *Candida* growth and the trial had to be discontinued for this reason after 10 d. This was not expected from the *in vitro* studies performed with the same mouthrinse, in which both *Candida* and *Streptococcus mutans* were inhibited and no inhibition of *Lactobacilli* was observed.

### References

Andersen O M. Chromatographic separation of anthocyanins in cowberry (lingonberry) Vaccinium vitis-idaea L., Journal of Food Science. 1985;50(5):1230-1232.
Blatherwick N.R. & Long M. L. The increased acidity produced by eating prunes and cranberries. Jour. Biol. Chem. 57:815-818. 1923.
Deubert K H. A rapid method for extraction and quantitation of total anthocyanin of cranberry fruit. Journal of Agricultural and Food Chemistry. 1978;26(6):1452-1453.
Frerichs G. An Improved Process of Manufacturing Vegetable Extracts Similar to Meat Extract. GB 191407262 A (30.11.0002) 1914.
Geissman TA (Ed). The chemistry of flavonoid compounds; Pergamon Press: 1962.
Griebel C. Beiträge zur Kenntnis der chemischen Zusammensetzung der Preiselbeeren, Moosenbeeren und Kranbeeren. Zeitschr. Untersuch. Nahr. und Genussmtl. 19: 241-252, 1910.
Kähkönen M P, Heinämäki J, Ollilainen V, Heinonen M. Berry anthocyanins: Isolation, identification and antioxidant activities. Journal of the Science of Food and Agriculture. 2003;83(14):1403-1411.
Laitinen L A, Tammela P S M, Galkin A, Vuorela H J, Marvola M L A, Vuorela P M. Effects of extracts of commonly consumed food supplements and food fractions on the permeability of drugs across caco-2 cell monolayers. Pharmaceutical Research. 2004;21(10):1904-1916.
Liu X, Xiao G, Chen W, XU Y, Wu J. Quantification and purification of mulberry anthocyanins with macroporous resins. Journal of Biomedicine and Biotechnology. 2004(5):326-331.
Mason G.C. The occurrence of benzoic acid naturally in cranberries. Jour. Amer. Chem. Soc. 27; 613-614. 1905.
Nelson E.K. Non-volatile acids of the pear, quince, apple, loganberry, blueberry, cranberry, lemon and pomegranate. Jour. Amer. Chem. Soc. 49; 1300-1302. 1927.
Radin M. J. A note of the quantity of benzoic acid contained in prunes and cranberries. Ind. & Eng. Chem. 6: 518.1914.
Rauha J-P, Remes S, Heinonen M, Hopia A, Kähkönen M, Kujala T, Pihlaja K, Vuorela H, Vuorela P. Antimicrobial effects of Finnish plant extracts containing flavonoids and other phenolic compounds. International Journal of Food Microbiology. 1999;56(1):3-12.
Swartz J H and Medrek T F. Antifungal properties of cranberry juice. Applied Microbiology. 1968;16(10):1524-1527.
Újváry I, Orlik J, Racz G, Donath A. On the fungistatic effect of cranberry's (Vaccinium vitis-idaea L.) crop-extract. (translation). Orv. Szemle. 1961;4:406-409.

## Claims

1. A lingonberry preparation manufactured by a process which comprises the steps of
a) fermenting cold-pressed lingonberry juice with *Saccharomyces cerevisiae,*
b) removing yeast cells from the fermented juice,
c) drying the juice to obtain a substantially dry cake,
d) milling the cake obtained into powder, and
e) pressing the powder into sucking tablets,
for use in inhibiting the growth of undesirable yeasts and bacteria and promoting the growth of probiotic bacteria in mouth.

2. The lingonberry preparation for use according to claim 1, wherein the moisture content of the cake is 1 - 5 %.

3. The lingonberry preparation for use according to claim 1, wherein the undesirable yeasts are of the genus *Candida,* and the undesirable bacteria are *Streptococci.*

4. The lingonberry preparation for use according to claim 1, wherein the probiotic bacteria are lactobacilli.

## Patentansprüche

1. Preiselbeerzubereitung, hergestellt durch ein Verfahren, das die folgenden Schritte umfasst:
a) Fermentieren von kaltgepresstem Preiselbeersaft mit *Saccharomyces cerevisiae,*
b) Entfernen von Hefezellen aus dem fermentierten Saft,
c) Trocknen des Safts, um einen im Wesentlichen trockenen Kuchen zu erhalten,
d) Mahlen des erhaltenen Kuchens zu Pulver, und
e) Pressen des Pulvers zu Lutschtabletten,
zur Anwendung bei der Hemmung des Wachstums unerwünschter Hefen und Bakterien und Förderung des Wachstums probiotischer Bakterien im Mund.

2. Preiselbeerzubereitung zur Anwendung nach Anspruch 1, wobei der Feuchtigkeitsgehalt des Kuchens 1 - 5 % beträgt.

3. Preiselbeerzubereitung zur Anwendung nach Anspruch 1, wobei die unerwünschten Hefen zur Gattung *Candida* gehören und die unerwünschten Bakterien *Streptococci* sind.

4. Preiselbeerzubereitung zur Anwendung nach Anspruch 1, wobei die probiotischen Bakterien Lactobacilli sind.

## Revendications

1. Préparation d'airelle fabriquée par un procédé qui comprend les étapes consistant à:
a) fermenter le jus d'airelle pressé à froid avec *Saccharomyces cerevisiae,*
b) éliminer les cellules de levure du jus fermenté,
c) sécher le jus pour obtenir un gâteau sensiblement sec,
d) broyer le gâteau obtenu en poudre, et
e) presser la poudre dans des comprimés à sucer,
pour une utilisation dans l'inhibition de la croissance de levures et de bactéries indésirables et la promotion de la croissance de bactéries probiotiques dans la bouche.

2. Préparation d'airelle pour une utilisation selon la revendication 1, dans laquelle la teneur en humidité du gâteau est de 1-5%.

3. Préparation d'airelle pour une utilisation selon la revendication 1, dans laquelle les levures indésirables sont du genre *Candida,* et les bactéries indésirables sont des *streptocoques.*

4. Préparation d'airelle pour une utilisation selon la revendication 1, dans laquelle les bactéries probiotiques sont des lactobacilles.
